(19) **Europäisches Patentamt / European Patent Office / Office européen des brevets**

(11) **EP 4 479 009 B1**

(12) **EUROPEAN PATENT SPECIFICATION**

(45) Date of publication and mention
of the grant of the patent:
**22.07.2026 Bulletin 2026/30**

(21) Application number: **22710527.7**

(22) Date of filing: **18.02.2022**

(51) International Patent Classification (IPC):
*A61F 13/15* (2006.01)  *A61F 13/511* (2006.01)
*A61F 13/512* (2006.01)  *A61F 13/513* (2006.01)
*A61F 13/84* (2006.01)

(52) Cooperative Patent Classification (CPC):
**A61F 13/15268; A61F 13/51121; A61F 13/512;
A61F 13/51394;** A61F 2013/15276;
A61F 2013/51139; A61F 2013/8497

(86) International application number:
**PCT/EP2022/054056**

(87) International publication number:
**WO 2023/155997 (24.08.2023 Gazette 2023/34)**

(54) **ABSORBENT ARTICLE**

ABSORBIERENDER ARTIKEL

ARTICLE ABSORBANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**25.12.2024 Bulletin 2024/52**

(73) Proprietor: **Essity Hygiene and Health Aktiebolag
405 03 Göteborg (SE)**

(72) Inventors:
• **PALMQVIST, Lisa
405 03 GÖTEBORG (SE)**

• **KNÖS, Anna
405 03 GÖTEBORG (SE)**
• **BLOMSTRÖM, Philip
405 03 GÖTEBORG (SE)**

(74) Representative: **Hoffmann Eitle
Patent- und Rechtsanwälte PartmbB
Arabellastraße 30
81925 München (DE)**

(56) References cited:
**EP-A1- 3 954 349      WO-A1-2021/224832
US-A1- 2016 113 826    US-A1- 2021 338 492**

**Description**

TECHNICAL FIELD

The disclosure relates to a washable and reusable absorbent undergarment as well as a washable and reusable absorbent assembly.

BACKGROUND

An absorbent article is worn for the purpose of absorbing body fluids such as urine and vaginal fluids. A washable and reusable absorbent undergarment comprises fabrics of woven or knitted materials. Conventionally such an absorbent undergarment comprises an integral absorbent assembly located in the crotch region of the wearer when the garment is worn. After use the absorbent undergarment is washed or laundered before reuse. The reusable absorbent undergarments have the look and feel of regular underwear. However, this may give the user a feeling of insecurity regarding the reliability of the absorbent undergarment and its ability to absorb the discharged body fluids. US 2016/0113826 A1 discloses a disposable absorbent article in which an apertured topsheet overlies a darker layer in order to give the impression of relative absorbency or breathability.

SUMMARY

The present disclosure is based on the insight that there is a need for a washable and reusable undergarment able to handle and absorb body fluids without substantial leakages while giving the user confidence in the reliability of the undergarment's absorbent capacity.
The present invention provides a washable and reusable absorbent assembly according to claim 1 and a washable and reusable absorbent undergarment according to claim 2. The dependent claims define particular embodiments and implementations of the invention.
The washable and reusable absorbent undergarment of knitted or woven material(s) has an extension in the longitudinal direction and in the transversal direction and comprises an absorbent assembly. The absorbent assembly comprises a first wearer facing layer having openings therein, a garment facing moisture barrier, a second layer directly beneath said top layer, the second layer being a wicking layer or an absorbent layer. The top layer has, in the CIE L* a* b* colour space, an L* value, the second layer has, in the CIE L* a* b* colour space, an L* value, and there is a mutual colour difference ΔE*ab between the top layer and the second layer of at least 15.
The colour difference between the top layer and the second layer directly beneath said top layer, i.e. an absorption layer, or a wicking layer in case such a layer is part of the absorbent assembly, makes the layer beneath the top layer visible through the openings in the top layer and thereby visualizing to the user the depth created by the openings in the top layer giving the user a confidence of the absorption capacity of the absorbent assembly.
The mutual colour difference ΔE*ab between the top layer and the second layer may be at least 25, such as at least 35, such as at least 50, such as at least 60.
The wicking layer or the absorbent layer may have a higher L* value than the top layer. By having a light(er) coloured layer beneath the top layer and which is visible through the openings in the top layer, the user may be given an estimation of the amount of absorbed menstrual fluid, or a sense of the fullness of the absorbent assembly. The visualization of the staining of the layer beneath the top layer also indicates to the user when the article needs to be exchanged and washed.
The second layer may have an L* value of more than 50 and the top layer may have an L* value of less than 50.
Due to the openings in the top layer, the absorbent assembly may receive a high amount of fluid in a short time without feeling overly wet. The wells will fill up with excess fluid that would otherwise flood the surface and thus give an uncomfortable, wet feeling. The filled-up wells will be emptied continuously to the layer directly beneath the top layer with openings. A high wicking and spreading capacity improve the fluid handling even further. The fluid is distributed across the layer for quick absorption and containment.
The openings in the top layer may each have dimensions within the range of 0.3-3.0 mm, such as 0.5-2.0 mm, such as 0.6-1.3 mm.
The top layer may have 10-50 openings/cm$^2$, such as 15-40 openings/cm$^2$, such as 15-25 openings/cm$^2$.
The openings may be arranged in rows. The rows may be transversal rows. The openings in adjacent rows may be offset in relation to each other. A "diamond" shaped macro pattern, i.e. the openings in a first row is offset with the openings in a second row adjacent the first row, such that each opening in the first row is situated centrally between two openings of the second row may give better directional rewet properties, more even spreading and less wetting of each row than parallel rows of holes only. If one or more holes become clogged in one direction, there is also a higher risk of leakage due to "flooding" in that direction if the holes are in parallel rows compared with a diamond shaped hole pattern.
Another feature of the diamond or offset pattern is less sensitivity towards ripping in x and y directions leading to a higher durability than an even hole pattern due to fewer holes per length unit.
The openings may be non-circular. The openings may have an elongated shape. The openings may be elongated and have a ratio of length to width of >1, such as >1.5, and such as <10, such as <5. A non-circular shape, such as oval, triangular or bird's eye shaped openings may direct the flow of body liquids in different directions within the absorbent assembly.

The top layer may be of a single layer. The top layer may not be a spacer fabric. The top layer may be of a Jacquard technique. The top layer may have a basis weight of 80-200 gsm. The top layer may be of polyester, elastane or blends thereof.

The top layer may be a weft knit, warp knit, flat knit or circular knit. The top layer may have 2-10 wales and 2-10 courses between openings. The openings may be tuck stitches. The openings may span 1-8 stitches, such as 2-6 stitches, such as 2-3 stitches. To benefit from a fast inlet the openings should be large enough to serve as wells, together with the layer directly beneath the top layer, i.e. two or more tuck stitches next to each other in a row or in two rows after each other in the course, may be beneficial.

The basis weight of the wicking layer may be 180-250 gsm. The wicking layer may be of polyester, polyamide, elastane or a mixture thereof. The wicking layer may be a jersey knit. The absorbent assembly may comprise two, three or four absorbent layers. The basis weight of an absorbent layer may be 200-350 gsm. The absorbent layer may be a hydrophilic French terry material and may be of polyester, polyamide, elastane or a mixture thereof.

The absorbent undergarment may comprise a body fabric. The fabric may define a waist opening, two leg openings and a crotch region between the leg openings. The absorbent assembly may be permanently attached to the fabric.

In a second aspect there is provided an absorbent assembly which may be used for and as a washable and reusable absorbent pad or napkin in a normal underwear. The features and advantages as set out in the above for the absorbent undergarment are equally applicable to the absorbent assembly.

BRIEF DESCRIPTION OF THE DRAWINGS

The disclosure will be described in greater detail below with reference to the figures shown in the appended drawings, wherein

Fig. 1    shows a top view of an absorbent undergarment with an absorbent assembly between the leg openings;

Fig. 2    shows a close-up of a top layer with openings;

Fig. 3    shows a front view of an absorbent undergarment;

Fig. 4    shows a cross-sectional view of a portion of an absorbent undergarment not including a wicking layer;

Fig. 5    shows a cross-sectional view of a portion of an absorbent undergarment including a wicking layer.

Fig. 6    shows a Table with ΔE*ab measurements of combinations of top layers and absorbent layers having different colour combinations.

DETAILED DESCRIPTION

Different aspects of the present disclosure will be described more fully hereinafter with reference to the enclosed drawings. The embodiments of the absorbent undergarment disclosed herein can, however be realized in many different forms, such as different sizes, colours and absorption levels, and should not be construed as being limited to the aspects set forth herein. In all figures in the following detailed description, the same reference numerals will be used to indicate the same elements.

As used herein, the term "absorbent undergarment" refers to garments that are worn and intended to be placed against the skin of the wearer to absorb and contain body fluids such as urine and vaginal fluids including menstrual fluid. The absorbent undergarment is an underwear of a fabric for female or male use. The undergarment according to the present disclosure is intended to be laundered or otherwise restored after use for reuse as a sanitary article. The absorbent assembly may be used as and/or for a washable and reusable absorbent pad or napkin.

By "laundering" it is meant that the absorbent undergarment may be cleaned by laundering. The absorbent undergarment may thus be subjected to an aqueous solution containing detergent without losing its structural features. This aqueous solution may be heated as part of the laundering process, e.g. to 40°C or 60°C.

The term "fabric" as used in the present disclosure may refer to single or multiple layers of fabrics. The fabric may be knitted or woven.

By "permanently attached", it is meant that the absorbent assembly is not intended to be separated from the body fabric before, during, or after use. The absorbent assembly as proposed herein is not necessarily directly bonded to the fabric, but instead may be attached via the leg opening seams or bonded by other means such as sealing tape. The absorbent assembly per se as proposed herein may or may not be permanently attached to an undergarment fabric material.

Colour is determined within the L*a*b* colour space, as established by the Commission Internationale de l'Éclairage (CIE) in 1976. The colour space is divided into three axes. L* represents lightness and the axis extends from 0 (black) to 100 (white). The axis a* goes from green to red, where positive values indicate more saturated red, and negative values more saturated green. The b*-axis goes from blue to yellow, where positive values represent more saturated yellow and negative values more saturated blue. This colour space is well known in industry and is generally referred to as CIE L*a*b* or CIELAB (1976). A difference between two colours in the colour space CIE L*a*b* is characterised by a Delta E-value (ΔE*ab). The differences between the colours on the three respective axes are squared in this, following which the differences are summed and the root derived from the sum. Substantially the same colours have a mutual colour difference ΔE*ab of less than 10.

Knitting is a method of constructing a fabric by interlocking a series of loops of one or more yarns. There are two major classes of knitting namely warp and weft knitting.

Weaving is a method or process of interlacing two yarns of similar materials so that they cross each other at right angles to produce woven fabric. The warp yarns run lengthwise in the fabric and the filling threads (weft) or picks, run from side to side. Jacquard is a system of weaving or knitting that utilizes a versatile pattern mechanism to produce intricate designs by using punch cards controlling the patterns produced. Jacquard knit may be valid for circular, flatbed, warp or weft knit. Openings or holes created by a knitting technique is generally referred to as tuck stitches herein.

The top layer may be constructed of any suitable fabric, including naturally derived fibers selected from the group consisting of cotton, wool, silk, cellulose, regenerated cellulose, rayon, viscose, modal, lyocell, Tencel, bamboo, hemp, flax, ramie, coir or banana. Alternatively, the top layer may be constructed of synthetic fibers selected from the group consisting of polyamide, acrylic, polyester or elastane, such as a mixture of polyester and elastane. Further, the top layer may be constructed of a blend or a mixture of naturally derived and/or synthetic fibers. The materials used for construction of the top layer should be soft and nonirritating to the skin and be readily penetrated by any body fluids. According to the present disclosure, the top layer should be launderable. The top layer may comprise between 20% and 100% naturally derived fibers, more preferably between 40% and 100% naturally derived fibers and most preferably between 60% and 100% naturally derived fibers. The top skin-facing layer comprises a water-permeable material thus allowing the body fluids to migrate to the underlying absorbent layer or layers. The top layer may have a basis weight of 80-200 gsm.

The top layer has openings therein. The openings may be non-circular. The openings may have an elongated shape. The openings may be elongated and have a ratio of length to width of >1, such as >1.5, and such as <10, such as <5. Non-circular openings, such as oval, triangular or bird's eye shaped openings may direct the flow of body liquids in different directions within the absorbent assembly. The openings in the top layer may each have dimensions within the range of 0.3-3.0, such as 0.5-2.0 mm, such as 0.6-1.3 mm.

The dimension of the openings may be measured in a conventional light microscope or stereo magnifier equipped with a ruler or image analysis. The material is laid out flat without stretching the material and the opening dimension is measured from one side of the opening to the opposite side of the opening. The dimensions in two crossing directions of an opening should be within the range mentioned herein. In case the opening span more than 1 stitch, such as 2 stitches, i.e. more than 1 adjacent tuck stitch, the opening dimension should be measured across the opening made up of the 2 combined tuck stitches.

A wicking layer may be provided underneath the top layer. The wicking layer has wicking features to allow the moisture to spread away from the wearer and into the absorbent layer or layers. Further, wicking enables an efficient spread of the moisture therefore allowing the moisture to be received in a wider area of the underlying one or more absorbent layers. This feature is particularly relevant for users suffering from stress incontinence as spurts of urine may cause the absorbent layer to be saturated rapidly at the point of impact. By spreading the volume of the fluid over a wider receiving area in the absorbent layer, the wicking features of the wicking layer increase the overall absorptive capacity of the absorbent layer. The wicking layer may be constructed of any suitable fabric, including naturally derived fibers or mixtures thereof selected from the group consisting of cotton, wool, silk, cellulose, regenerated cellulose, rayon, viscose, modal, lyocell, Tencel, bamboo, hemp, flax, ramie, coir or banana. Alternatively, the wicking layer may be constructed of synthetic fibers or mixtures thereof selected from the group consisting of polyamide, acrylic, polyester or elastane. Further, the wicking layer may be constructed of a blend or a mixture of naturally derived and/or synthetic fibers. According to the present disclosure, the wicking layer should be launderable. The wicking layer may comprise between 20% and 100% naturally derived fibers, more preferably between 40% and 100% naturally derived fibers and most preferably between 60% and 100% naturally derived fibers. The basis weight of the wicking layer may be 180-250 gsm.

The absorbent assembly for use in an absorbent undergarment may contain one or multiple absorbent layers, capable of absorbing liquid and releasing the liquid during laundering. Release of the absorbed liquid may be beneficial as it enables the absorbent undergarment to be restored for reuse. Reusable absorbent undergarments may provide a more sustainable alternative to the commonly used disposable hygiene articles that are not intended to be re-used.

The absorbent layer may comprise any material capable of absorbing fluid, such as woven or nonwoven microfiber or polymer knits, fabric formed from hydrophilic fibers, absorbent fibers or powders. The absorbent layer may be of natural or synthetic fibers as described above for the other ingoing layers but may be of polyester and polyamide and containing odor treatment.

The absorbent layer may also comprise a material having an open cell porous structures such as high loft or synthetic fibers having reservoir properties. The absorbent layer may comprise between 20% and 100% naturally derived fibers, more preferably between 40% and 100% naturally derived fibers and most preferably between 60% and 100% naturally derived fibers. The basis weight of each absorbent layer may be 200-350 gsm. There may be one or more, such as 2, absorbent layers in the absorbent assembly.

The liquid-impermeable barrier layer may comprise any suitable material or combinations of material that prevents liquid from migrating from the absorbent assembly to the fabric layer. The liquid-impermeable barrier layer may comprise a hydrophobic woven or nonwoven material having inherent hydrophobic properties, or that has been treated to become hydrophobic. Examples of hydrophobic materials for treating the barrier layer are polymers such as silicone, polyurethane and combinations thereof. The liquid-impermeable barrier layer may comprise a microporous polymer film, e.g. a polyethylene, PTFE or polyurethane film, or combinations thereof. Laminates of polymerr films and nonwoven materials may also be used. The liquid-impermeable barrier layer may be a coating of a moisture-impermeable material. The coating may be a polymer such as urethane wax on the surface facing away from the wearer. The liquid-impermeable barrier layer may be of polyurethane.

The liquid-impermeable barrier layer is preferably breathable, to allow vapor to escape from the absorbent undergarment, while preventing liquid from passing through the fabric layer. Further, the liquid-impermeable barrier layer may be constructed of an elastic material, thus providing the absorbent assembly with the required flexibility to adapt to the user's anatomy and movements. This improved fit increases the wearer's comfort during use and helps to prevent leakage from migrating through the undergarment's leg openings.

The absorbent undergarment comprises a body fabric. The fabric may define a waist opening, two leg openings and a crotch region between the leg openings. The absorbent assembly may be permanently attached to the body fabric. The fabric of the undergarment may be constructed of any suitable fabric, including naturally derived fibers and mixtures thereof selected from the group consisting of cotton, wool, silk, cellulose, regenerated cellulose, rayon, viscose, modal, lyocell, Tencel, bamboo, hemp, flax, ramie, coir or banana. Alternatively, the fabric may be constructed of synthetic fibers or mixtures thereof selected from the group consisting of polyamide, acrylic, polyester. Further, the fabric may be constructed of a blend or a mixture of naturally derived and/or synthetic fibers. The fibers may be recycled fibers. The fabric may comprise a stretchable fabric, e.g. elastane, so that the absorbent undergarment can provide a firm fit while at the same time adapting to the wearer's movements thus preventing any leakage from migrating through the leg openings and keeping the absorbent assembly in place. The fabric is preferably breathable to allow vapor to escape from the wearer's skin and from the absorbent structure.

The layers of the absorbent assembly and the undergarment body fabric may be bonded by conventional stitching, gluing techniques or via a bonding film such as tape.

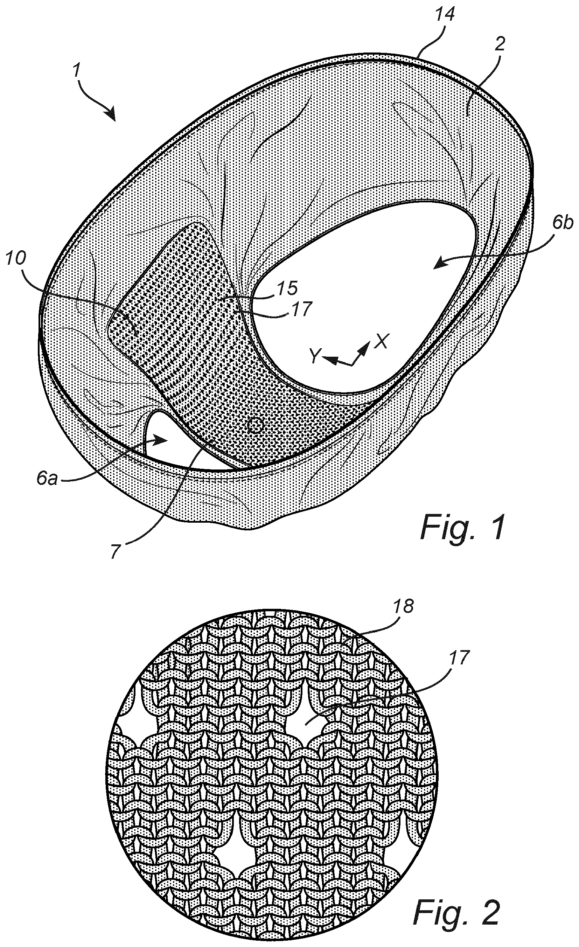

Fig. 1 discloses a washable and reusable absorbent undergarment 1 in the form of a panty comprising a fabric 2 defining a waist opening, two leg openings 6a, 6b and a crotch region 7 between the leg openings 6a, 6b. The absorbent undergarment 1 further comprises an absorbent assembly 10 covering at least part of the crotch region 7 and is permanently attached to the fabric layer 2. The absorbent assembly 10 comprises a knitted skin-facing top layer 15. The top layer 15 has openings 17 therein, each opening may span 1-10 stitches and there may be 5-60 openings/cm$^2$. The top layer may be a single layer jacquard stitched material and may have a basis weight of 80-200 gsm. The openings 17 may be arranged in transversal rows. The second layer, i.e. the wicking layer 16 or an absorbent layer 11 has an L* value, the top layer 17 has an L* value, and there is a mutual colour difference $\Delta E^*ab$ between the top layer 17 and the second layer 16, 11 of at least 15.

One embodiment for feminine use may have a top layer 15 of a jacquard knit having elongated openings 17 therein that span two stitches, i.e. 2 tuck stitches, and have 20 openings/cm$^2$ and each stitch having a largest dimension of about 1.1 mm and a smallest dimension of about 0.8 mm. Such a solution has been shown to be useful for heavy period flows. Another embodiment may have a top layer 15 having openings 17 therein that span three stitches and have 50 openings/cm$^2$ and each opening having a largest dimension of about 1.5 mm and a smallest dimension of about 0.5 mm. Such a solution has been shown to be useful for incontinence use where large volumes of urine needs to be taken care of.

Figure 2 discloses a close-up of a knitted top layer 15 with the threads 18 and openings 17. The openings are arranged in rows, wherein the openings 17 in adjacent transversal rows are offset in relation to each other. Each opening is of 1 tuck stitch. The top layer 15 has a lower L* value than the second layer 16, 11 which is visible through the openings 17 in the top layer 15.

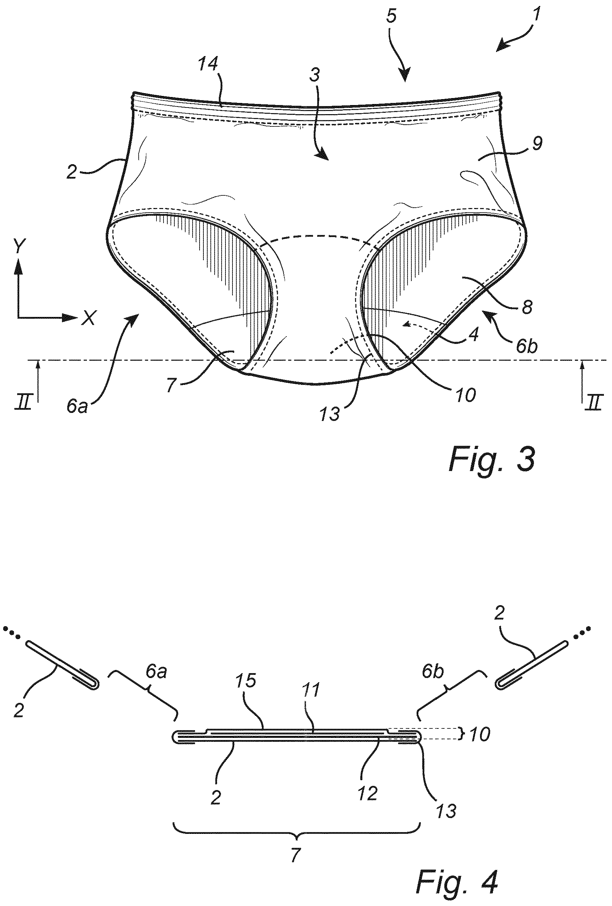

Figure 3 is a front view of an absorbent undergarment 1 comprising a fabric layer 2 having a front side 3, a back side 4 and a waist opening 5. The fabric layer 2 has two leg openings 6a and 6b, and a crotch region 7 in between the leg openings 6a and 6b. The fabric layer 2 further includes an interior surface 8, i.e. a surface of the fabric layer 2 that wholly or partially contacts the body of the wearer, and an exterior surface 9, i.e. a surface of the fabric layer 2 facing away from the wearer. The absorbent undergarment 1 further comprises an absorbent assembly 10 covering at least part of said crotch region 7. In figure 3, a leg opening seam 13 lines the leg openings 6a and 6b. The leg opening seam 13 may be provided by conventional stitching or gluing techniques. The leg opening seam 13 may comprise an elastic member such as elastic bonding film of polyurethane. Further, the leg opening seam 13 may serve to bond the layers of the absorbent assembly 10, including the top layer 15, the absorbent layer(s) 11, the outer liquid impermeable barrier layer 12 and the wicking layer 16, together. The absorbent undergarment 1 may further comprise a waist band 14. The leg opening and the absorbent assembly may be sealed with a tape (not shown) to avoid side leakage at the leg openings.

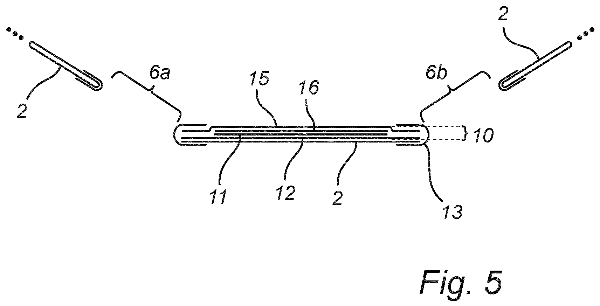

Figure 4 shows a cross-sectional view of the crotch region 7 of an absorbent undergarment 1. The absorbent undergarment 1 comprises the absorbent assembly 10, arranged on the interior surface 8 of the fabric layer 2 and covering a least part of the crotch region 7. The absorbent assembly 10 may comprise one or more absorbent layers 11. In figures 4 and 5, only one absorbent layer 11 is shown to increase clarity of the figures. Further, the absorbent assembly 10 comprises a liquid-impermeable barrier or barrier layer 12 located between the one or more absorbent layers 11 and the fabric layer 2. The liquid-impermeable barrier 12 prevents liquid from leaking through the one or more absorbent layers 11 into the fabric layer 2. The liquid-impermeable barrier 12 may be a coating on the wearer facing side of the fabric layer 2 or on a garment facing side of the absorbent layer 11. The absorbent assembly 10 further comprises a top layer 15 facing the skin of the user. The different layers of the absorbent assembly 10, but additionally also the fabric layer 2, are in Figure 4 bonded together via the leg opening seam 13.

Figure 5 shows an absorbent undergarment 1 wherein the absorbent assembly comprises a wicking layer 16 directly beneath the skin-facing top layer 15 and in contact with the absorbent layer 11. A liquid-impermeable barrier 12 is located between the absorbent layer 11 and the undergarment fabric 2.

Examples Colour measurement

Colour difference (ΔE*ab) between the top layer and an underlying absorbent layer or wicking layer is determined within the CIE L* a* b* (1976) colour space. The colours are measured with a spectrophotometer. A suitable instrument can be SpectroDens Premium from Techkon GmbH (Köningstein, Germany) or equivalent. The instrument has a measurement geometry of 0/45° (according to **DIN** 5033). The viewing angle is set to 2° and illuminant D50 is selected. Any polarizing filter should be inactivated. Before measurement, the instrument is calibrated according to instructions from the manufacturer. Readings are made through an aperture with a 3 mm diameter.

The relevant layers are carefully separated from the absorbent undergarment. The layer for testing is then placed flat and smooth, but not stretched, on top of a standard background of three A4 sheets of conventional office printing paper (80 gsm per sheet). The stack of three sheets has values L*= 95.5, a*= 2.2 and b= -6.5 as measured with the spectrophotometer settings specified above. Measurements are made on the side of the layer that faces upwards towards the user in the absorbent garment. Sites for measurement are selected such that the openings in the sample layer are avoided to the extent possible, and such that no wrinkles, embossings or material irregularities unduly interfere. In case the sample layer comprises more than one colour, all measurements are performed on the most representative colour, i.e. the single colour that covers the largest proportion of the layer. Ten different sites are measured for each layer, and arithmetic mean values are calculated for L*, a* and b*.

The mean values from the two compared layers are then put into the ΔE* calculation. In this, the differences between the two layers on each of the axis L*, a* and b* are squared, summed up, and the root is then taken from the sum:

$$\Delta E^*ab = \sqrt{(\Delta L^*)^2 + (\Delta a^*)^2 + (\Delta b^*)^2}$$

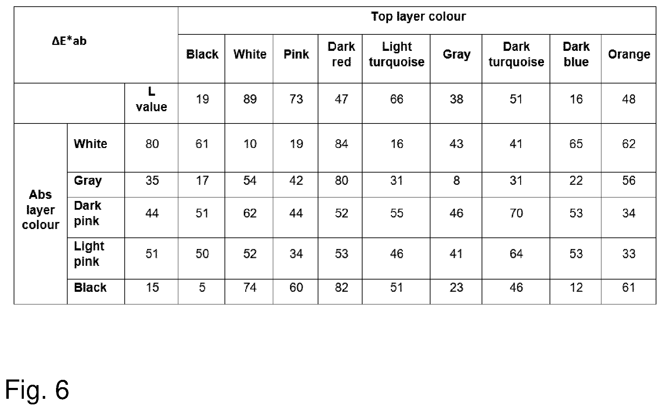

The result of the colour measurements of the top layer and the layer beneath, in this case the absorbent layer, is shown in the Table in Fig. 6. The colours of the tested absorbent layers are shown in the second column on the left-hand side.

The L values for the tested absorbent layers are shown in the third column from the left. The colours of the tested top layers are shown in the second horizontal row. The L values for the tested top layers are shown in the third row.

The ΔE*ab values for the different combinations of top layer colours and absorbent layer colours are shown from the fourth to eighth row in the fourth to 12th column.

From the result of the measurements in Fig. 6 it may be concluded that there need to be a colour difference ΔE*ab between the top layer and the layer beneath (the absorbent layer in the examples) of at least 15 to obtain the desired effect of visualizing the depth of the openings and/or the absorbent capacity of the absorbent assembly.

The disclosure may be varied within the scope of the appended claims. For example, the materials and dimensions used for the different layers forming the absorbent insert may be varied, as indicated above.

**Claims**

1. A washable and reusable absorbent assembly (10) comprising a first wearer facing layer (15) having openings (17) therein, a garment facing moisture barrier (12), a second layer (16, 11) directly beneath said wearer facing layer (15), the second layer (16, 11) being a wicking layer (16) arranged above an absorbent layer or itself being an absorbent layer (11), wherein the wearer facing layer (15) has, in the CIE L* a* b* colour space, an L* value, the second layer (16, 11) has, in the CIE L* a* b* colour space, an L* value, and there is a mutual colour difference ΔE*ab between the wearer facing layer (15) and the second layer (16, 11) of at least 15.

**2.** A washable and reusable absorbent undergarment (1) of knitted or woven material(s) having an extension in the longitudinal direction (y) and in the transversal direction (x) comprising an absorbent assembly (10)as recited in claim 1.

**3.** Undergarment according to claim 2, wherein colour difference ΔE*ab is at least 25.

**4.** Undergarment according to anyone of the claims 2 or 3, wherein colour difference ΔE*ab is at least 35.

**5.** Undergarment according to anyone of the claims 2-4, wherein the second layer (16, 11) has a higher L* value than the wearer facing layer (15).

**6.** Undergarment according to anyone of the claims 2-5, wherein the openings (17) in the wearer facing layer (15) each has a dimension within the range of 0.3-3 mm.

**7.** Undergarment according to anyone of the claims 2-6, wherein the wearer facing layer (15) is of a knitted fabric.

**8.** Undergarment according to anyone of the claims 2-7, wherein each opening (17) in the wearer facing layer (15) span 1-10 stitches and there are 5-60 openings/cm$^2$.

**9.** Undergarment according to any one of claims 2-8, wherein each opening (17) spans 1-8 stitches.

**10.** Undergarment according to any one of claims 2-9, wherein the openings (17) are arranged in transversal (x) rows.

**11.** Undergarment according to claim 10, wherein the openings (17) in adjacent rows are offset in relation to each other.

**12.** Undergarment according to any one of claims 2-11, wherein the wearer facing layer (15) is of a Jacquard technique.

**13.** Undergarment according to any one of claims 2-12, wherein the wearer facing layer (15) is a single layer.

**14.** Undergarment according to any one of claims 2-13, wherein the openings (17) are tuck stitches.

**15.** Undergarment according to any one of claims 2-14, wherein the absorbent assembly (10) comprises two absorbent layers (11).

**Patentansprüche**

**1.** Waschbare und wiederverwendbare absorbierende Anordnung (10), aufweisend eine erste dem Träger/-der Trägerin zugewandte Lage (15) mit Öffnungen (17) darin, eine dem Wäschestück zugewandte Feuchtigkeitsbarriere (12), eine zweite Lage (16, 11) direkt unter der dem Träger/der Trägerin zugewandten Lage (15), wobei die zweite Lage (16, 11) eine feuchtigkeitstransportierende Schicht (16) ist, die über einer absorbierenden Lage angeordnet oder selbst eine absorbierende Lage (11) ist, wobei die dem Träger/der Trägerin zugewandte Lage (15) im CIE L*a*b*-Farbraum einen L*-Wert hat, die zweite Lage (16, 11) im CIE L*a*b*-Farbraum einen L*-Wert hat, und ein gegenseitiger Farbabstand ΔE*ab zwischen der dem Träger/der Trägerin zugewandten Lage (15) und der zweiten Lage (16, 11) von mindestens 15 besteht.

**2.** Waschbare und wiederverwendbare absorbierende Unterwäsche (1) aus gestricktem oder gewebtem Material(ien), die sich in Längsrichtung (y) und in Querrichtung (x) erstreckt und eine absorbierende Anordnung (10) nach Anspruch 1 aufweist.

**3.** Unterwäsche nach Anspruch 2, wobei der Farbabstand ΔE*ab mindestens 25 beträgt.

**4.** Unterwäsche nach einem der Ansprüche 2 oder 3, wobei der Farbabstand ΔE*ab mindestens 35 beträgt.

**5.** Unterwäsche nach einem der Ansprüche 2-4, wobei die zweite Lage (16, 11) einen höheren L*-Wert als die dem Träger/der Trägerin zugewandte Lage (15) hat.

**6.** Unterwäsche nach einem der Ansprüche 2-5, wobei die Öffnungen (17) in der dem Träger/der Trägerin zugewandten Lage (15) jeweils eine Abmessung im Bereich von 0.3-3 mm haben.

**7.** Unterwäsche nach einem der Ansprüche 2-6, wobei die dem Träger/der Trägerin zugewandte Lage (15) aus einem Gestrick besteht.

**8.** Unterwäsche nach einem der Ansprüche 2-7, wobei jede Öffnung (17) in der dem Träger/der Trägerin zugewandten Lage (15) 1-10 Maschen überspannt und es 5-60 Öffnungen/cm$^2$ gibt.

**9.** Unterwäsche nach einem der Ansprüche 2-8, wobei jede Öffnung (17) 1-8 Maschen überspannt.

**10.** Unterwäsche nach einem der Ansprüche 2-9, wobei die Öffnungen (17) in Quer- (x) Reihen angeordnet sind.

**11.** Unterwäsche nach Anspruch 10, wobei die Öffnungen (17) in benachbarten Reihen zueinander versetzt sind.

**12.** Unterwäsche nach einem der Ansprüche 2-11, wobei die dem Träger/der Trägerin zugewandte Lage (15) mittels Jacquardtechnik hergestellt ist.

**13.** Unterwäsche nach einem der Ansprüche 2-12, wobei die dem Träger/der Trägerin zugewandte Lage (15) eine einfache Lage ist.

**14.** Unterwäsche nach einem der Ansprüche 2-13, wobei die Öffnungen (17) Fangmaschen sind.

**15.** Unterwäsche nach einem der Ansprüche 2-14, wobei die absorbierende Anordnung (10) zwei absorbierende Lagen (11) aufweist.

**Revendications**

**1.** Ensemble absorbant lavable et réutilisable (10) comprenant une première couche en contact avec un utilisateur (15), comportant des ouvertures (17), une barrière anti-humidité tournée vers un vêtement (12), une deuxième couche (16, 11) située directement sous la couche en contact avec un utilisateur (15), la deuxième couche (16, 11) étant une couche de transport d'humidité (16) disposée au-dessus d'une couche absorbante ou constituant elle-même une couche absorbante (11), dans lequel la couche en contact avec un utilisateur (15) présente, dans l'espace colorimétrique CIE L* a* b*, une valeur L*, la deuxième couche (16, 11) présente, dans l'espace colorimétrique CIE L* a* b*, une valeur L*, et il existe une différence de couleur mutuelle ΔE*ab entre la couche en contact avec un utilisateur (15) et la deuxième couche (16, 11) d'au moins 15.

**2.** Sous-vêtement absorbant lavable et réutilisable (1) de matière(s) tricotée(s) ou tissée(s) ayant une extension dans la direction longitudinale (y) et dans la direction transversale (x), comprenant un ensemble absorbant (10) tel que défini dans la revendication 1.

**3.** Sous-vêtement selon la revendication 2, dans lequel une différence de couleur ΔE*ab est d'au moins 25.

**4.** Sous-vêtement selon l'une quelconque des revendications 2 ou 3, dans lequel une différence de couleur ΔE*ab est d'au moins 35.

**5.** Sous-vêtement selon l'une quelconque des revendications 2 à 4, dans lequel la deuxième couche (16, 11) présente une valeur L* supérieure à la couche en contact avec un utilisateur (15).

**6.** Sous-vêtement selon l'une quelconque des revendications 2 à 5, dans lequel les ouvertures (17) dans la couche en contact avec un utilisateur (15) ont chacune une dimension comprise dans une plage de 0,3 à 3 mm.

**7.** Sous-vêtement selon l'une quelconque des revendications 2 à 6, dans lequel la couche en contact avec un utilisateur (15) est constituée d'un tissu tricoté.

**8.** Sous-vêtement selon l'une quelconque des revendications 2 à 7, dans lequel chaque ouverture (17) dans la couche en contact avec un utilisateur (15) s'étend sur 1 à 10 mailles et il y a de 5 à 60 ouvertures/cm2.

**9.** Sous-vêtement selon l'une quelconque des revendications 2 à 8, dans lequel chaque ouverture (17) s'étend sur 1 à 8 mailles.

**10.** Sous-vêtement selon l'une quelconque des revendications 2 à 9, dans lequel les ouvertures (17) sont disposées en rangées transversales (x).

**11.** Sous-vêtement selon la revendication 10, dans lequel les ouvertures (17) des rangées adjacentes sont décalées les unes par rapport aux autres.

**12.** Sous-vêtement selon l'une quelconque des revendications 2 à 11, dans lequel la couche en contact avec un utilisateur (15) est réalisée selon une technique Jacquard.

**13.** Sous-vêtement selon l'une quelconque des revendications 2 à 12, dans lequel la couche en contact avec un utilisateur (15) est une couche unique.

**14.** Sous-vêtement selon l'une quelconque des revendications 2 à 13, dans lequel les ouvertures (17) sont des mailles rabattues.

**15.** Sous-vêtement selon l'une quelconque des revendications 2 à 14, dans lequel l'ensemble absorbant (10) comprend deux couches absorbantes (11).

*Fig. 1*

*Fig. 2*

Fig. 3

Fig. 4

*Fig. 5*

| ΔE*ab | | | Top layer colour | | | | | | | | |
|---|---|---|---|---|---|---|---|---|---|---|---|
| | | | Black | White | Pink | Dark red | Light turquoise | Gray | Dark turquoise | Dark blue | Orange |
| | | L value | 19 | 89 | 73 | 47 | 66 | 38 | 51 | 16 | 48 |
| Abs layer colour | White | 80 | 61 | 10 | 19 | 84 | 16 | 43 | 41 | 65 | 62 |
| | Gray | 35 | 17 | 54 | 42 | 80 | 31 | 8 | 31 | 22 | 56 |
| | Dark pink | 44 | 51 | 62 | 44 | 52 | 55 | 46 | 70 | 53 | 34 |
| | Light pink | 51 | 50 | 52 | 34 | 53 | 46 | 41 | 64 | 53 | 33 |
| | Black | 15 | 5 | 74 | 60 | 82 | 51 | 23 | 46 | 12 | 61 |

# Fig. 6

**EP 4 479 009 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20160113826 A1